(19) Europäisches Patentamt
European Patent Office
Office européen des brevets

(11) Veröffentlichungsnummer: **0 002 671**
**A1**

(12) # EUROPÄISCHE PATENTANMELDUNG

(21) Anmeldenummer: **78101486.5**

(22) Anmeldetag: **01.12.78**

(51) Int. Cl.²: **C 07 D 249/08**, A 01 N 9/22

(30) Priorität: **29.12.77 DE 2758784**

(43) Veröffentlichungstag der Anmeldung: **11.07.79**
**Patentblatt 79/14**

(84) Benannte Vertragsstaaten: **BE CH DE FR GB IT LU NL SE**

(71) Anmelder: **BASF Aktiengesellschaft, Carl-Bosch-Strasse 38, D-6700 Ludwigshafen (DE)**

(72) Erfinder: **Zeeh, Bernd, Dr., Thorwaldsenstrasse 5, D-6700 Ludwigshafen (DE)**
Erfinder: **Rentzea, Costin, Dr., Neuenheimer Landstrasse 72, D-6900 Heidelberg (DE)**
Erfinder: **Pommer, Ernst-Heinrich, Dr., Berliner Platz 7, D-6703 Limburgerhof (DE)**

(54) **Substituierte Triazolylglykoläther, Verfahren zu ihrer Herstellung sowie ihre Verwendung als Fungizide.**

(57) Triazolyl-glykoläther der Formel

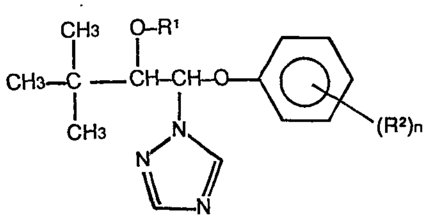

in der

$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluormethyl substituiert ist,
$R^2$ ein Halogenatom bedeutet und
n 1 oder 2 bedeutet

und deren Salze sowie Metallkomplexverbindungen, mit guter fungizider Wirkung, Fungizide die diese Verbindungen als Wirkstoffe enthalten, und Verfahren zur Bekämpfung von Pilzen mit diesen Verbindungen.

EP 0 002 671 A1

Triazolyl-glykoläther

BEZEICHNUNG GEÄNDERT
siehe Titelseite

Die vorliegende Erfindung betrifft wertvolle neue substituierte Triazolyl-glykoläther sowie deren Salze und Metallkomplexverbindungen mit guter fungizider Wirkung, Fungizide, die diese Verbindungen enthalten, Verfahren zu ihrer Herstellung und ihre Verwendung als Fungizide.

Es ist bekannt, Imidazol-substituierte Äther als Fungizide zur Bekämpfung pflanzenpathogener Pilze zu verwenden (DE-OS 20 63 857).

Es wurde gefunden, daß Triazolyl-glykoläther der Formel I

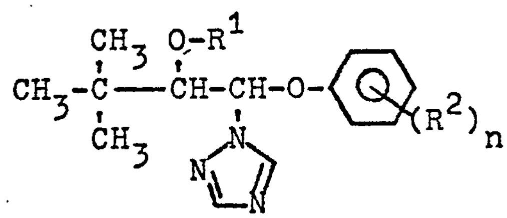

I

in der

$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluoromethyl substituiert ist,

$R^2$ ein Halogenatom bedeutet und

n 1 oder 2 bedeutet,

sowie deren Salze und Metallkomplexverbindungen eine sehr gute fungizide Wirksamkeit bei ausgezeichneter Pflanzenverträglichkeit zeigen. Salze sind z.B. die Hydrochloride, Hydrobromide, Sulfate, Oxalate, Nitrate oder Dodecylbenzolsulfonate.

Die Metallkomplexverbindungen haben die allgemeine Formel

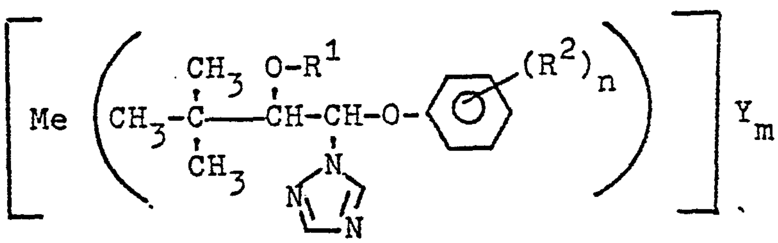 II

in welcher $R^1$, $R^2$ und n die oben angegebene Bedeutung haben, Me ein Metallkation bedeutet, z.B. ein Kation von Kupfer, Zink oder Zinn und Y das Anion einer anorganischen Säure, z.B. Salzsäure, Bromwasserstoffsäure, Schwefelsäure oder Salpetersäure und m eine Zahl von 1 bis 4 bedeutet.

Es wurde weiterhin gefunden, daß man die neuen Triazolylglykoläther der Formel I erhält, wenn man ein Triazolyl-Derivat der Formel

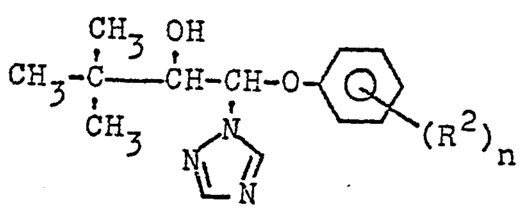 III

worin $R^2$ und n die oben angegebene Bedeutung haben

a) mit einem Halogen-Derivat der Formel

$$Hal-R^1 \qquad (IV)$$

in welcher
$R^1$ die oben angegebene Bedeutung hat, und
Hal Halogen, insbesondere Chlor, Brom oder Jod bedeutet oder

b) mit einem Sulfonsäureester der Formel

$$R^3-\overset{O}{\underset{O}{\overset{\|}{\underset{\|}{S}}}}-O-R^1 \qquad (V)$$

in welcher
$R^1$ die oben angegebene Bedeutung hat, und
$R^3$ $-O-R^1$, Phenyl, p-Tolyl oder p-Brom-phenyl bedeutet,

in Gegenwart eines Lösungsmittels und gegebenenfalls in Gegenwart eines basischen Katalysators und gegebenenfalls in Gegenwart eines Kronenäthers als weiterem Katalysator umsetzt.

Ferner können die erfindungsgemäß erhältlichen Triazolylglykoläther der Formel (I) durch Umsetzen mit Säuren in die Salze überführt werden, bzw. können durch Reaktion mit Metallsalzen die entsprechenden Metallkomplexe erhalten werden.

Verwendet man 1-(4-Chlorphenoxy)-1-[1,2,4-Triazolyl-(1)]-3,3-dimethyl-butan-2-ol und Benzylbromid als Ausgangsstoffe, so kann der Reaktionsablauf durch das folgende Formel-

schema wiedergegeben werden (Verfahren a):

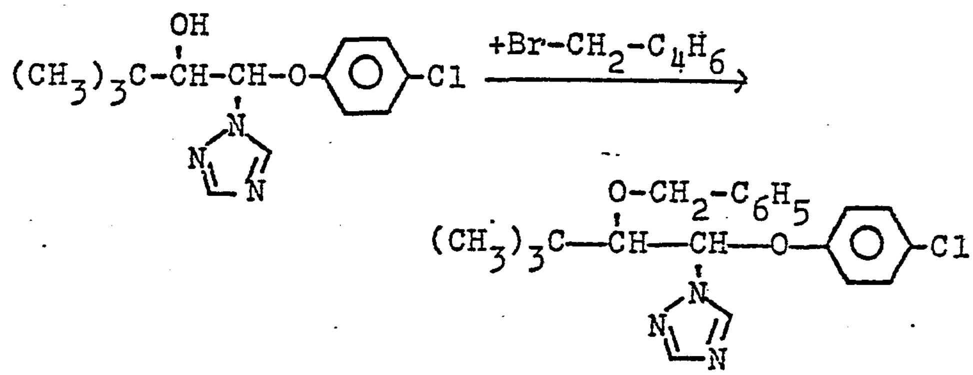


Verwendet man 1-(4-Bromphenoxy)-1-[1,2,4-Triazolyl-(1)]-3,3-dimethyl-butan-2-ol und Dimethylsulfat als Ausgangsstoffe, so kann der Reaktionsablauf durch das folgende Formelschema wiedergegeben werden (Verfahren b):

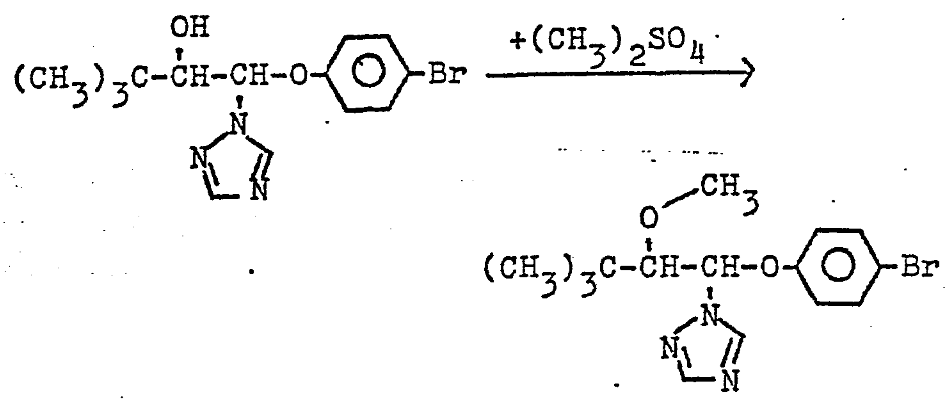


Die als Ausgangsstoffe verwendeten Triazolyl-Derivate sind durch die Formel (III) allgemein definiert (DE-OS 23 24 010).
Die für die Herstellung der erfindungsgemäßen Wirkstoffe gemäß Verfahrensvariante a) erforderlichen Halogen-Derivate sind durch die Formel (IV) allgemein definiert.

$R^1$ bedeutet beispielsweise einen geradkettigen oder verzweigten Alkylrest mit 1 bis 5, insbesondere 1 bis 3 Kohlenstoffatomen; einen geradkettigen oder verzweigten Alkenylrest oder einen Alkinylrest mit jeweils 3 bis 5 Kohlenstoffatomen oder einen gegebenenfalls durch Alkyl, Alkoxy, Halogen und Trifluormethyl substituierten Benzylrest.

Die Halogen-Derivate der Formel (IV) sind bekannt oder lassen sich nach üblichen Verfahren herstellen, die aus der Literatur allgemein bekannt sind.

Die weiteren für die Herstellung der erfindungsgemäßen Stoffe gemäß Verfahrensvariante b) erforderlichen Sulfate und Sulfonate sind durch die Formel (V) allgemein definiert.

Für die Herstellung der erfindungsgemäßen Wirkstoffe der Formel I gemäß Verfahrensvariante a) und b) sind beispielsweise Diäthyläther, Dibutyläther, Tetrahydrofuran, Dioxan, 1,2-Dimethoxyäthan oder ihre Gemische mit 18-Kronen-6-äther oder Dibenzo-18-kronen-6-äther oder Dimethylformamid, N-Methyl-pyrrolidon oder Hexamethyl-phosphor-triamid vorteilhafte Lösungsmittel. Als basische Katalysatoren können Lithiumhydrid, Natriumhydrid, Methylmagnesiumchlorid, Methylmagnesiumbromid, Butyllithium, Phenyllithium, Natriumnaphthalenid und Natriummethoxid eingesetzt werden. Die Reaktion wird am besten zwischen -80 bis +80°C, vorzugsweise zwischen 0 bis 60°C durchgeführt.

Für die Herstellung der Metallkomplexe der Formel II kommen alle mit Wasser mischbaren Lösungsmittel in Frage. Gut geeignet sind Methanol, Äthanol, Aceton und Tetrahydrofuran.

Als Beispiele für die neuen Triazolyl-glykoläther seien im einzelnen genannt:

2-Methoxy-1-(4'-chlorphenoxy)-1-[1,2,4-triazolyl-(1)]-3,3-dimethyl-butan

2-Äthoxy-1-(4'-chlorphenoxy)-1-[1,2,4-triazolyl-(1)]-3,3-dimethyl-butan

2-Allyloxy-1-(4'-chlorphenoxy)-1-[1,2,4-triazolyl-(1)]-3,3-dimethyl-butan

2-Propargyloxy-1-(4'-chlorphenoxy)-1-[1,2,4-triazolyl-(1)]-3,3-dimethyl-butan

2-(4'-Chlorbenzyloxy)-1-(4"-chlorphenoxy)-1-[1,2,4-triazolyl-(1)]-3,3-dimethyl-butan

2-(4'-Trifluormethyl-benzyloxy)-1-(4"-chlorphenoxy)-1-[1,2,4-triazolyl-(1)]-3,3-dimethyl-butan

2-(4'-Methoxy-benzyloxy)-1-(4"-chlorphenoxy)-1-[1,2,4-triazolyl-(1)]-3,3-dimethyl-butan

2-(2',4'-Dichlor-benzyloxy)-1-(4"-chlorphenoxy)-1-[1,2,4-triazolyl-(1)]-3,3-dimethyl-butan

2-(4'-Brombenzyloxy)-1-(4"-chlorphenoxy)-1-[1,2,4-triazolyl-(1)]-3,3-dimethyl-butan

2-Benzyloxy-1-(4'-chlorphenoxy)-1-[1,2,4-triazolyl-(1)]-3,3-dimethyl-butan

2- Methoxy-1-(4'-bromophenoxy )-1-[1,2,4-triazolyl-(1)]-3,3-dimethyl-butan

2-Allyloxy-1(4'-bromophenoxy)-1-[1,2,4-triazolyl-(1)]-3,3-dimethyl-butan

2-Methoxy-1-(2',4'-dichlorphenoxy)-1-[1,2,4-triazolyl-(1)]-3,3-dimethyl-butan.

Die Verbindungen der Formel (I) besitzen zwei chirale Kohlenstoffatome; sie können deshalb in der erytro-/threo-Form vorliegen. In beiden Fällen liegen sie vorwiegend als Racemate vor. Die erythro- und threo-Formen lassen sich trennen (z.B. durch Säulenchromatographie) und in reiner Form isolieren.

<u>Herstellungsbeispiele</u>

<u>Beispiel 1</u>

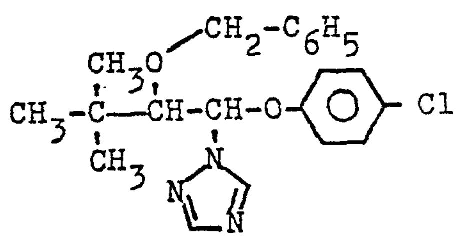

29,5 Teile (Gewichtsteile) 1-(4'-Chlorphenoxy)-1-[1,2,4-triazolyl-(1)]-3,3-dimethyl-butan-2-ol werden in 250 Teilen wasserfreiem Tetrahydrofuran gelöst. Dazu werden bei 10 - 15°C unter Stickstoff, Rühren und Eiskühlung in Portionen 3,4 Teile Natriumhydrid gegeben und 2 Stunden bei 10 bis 15°C, danach 12 Stunden bei Raumtemperatur gerührt. Anschließend wird auf 10°C abekühlt und bei 10 bis 20°C 19,7 Teile Benzylbromid zugetropft. Nach zwölfstündigem Rühren bei Raumtemperatur wird die entstandene Suspension vorsichtig mit 10 Teilen Wasser tropfenweise versetzt und im Wasserstrahlvakuum eingeengt. Der Rückstand wird in 200 Teilen Methylenchlorid gelöst. zweimal mit je 80 Teilen Wasser gewaschen, über $NaSO_4$ getrocknet und einge-

engt. Der Rückstand wird zweimal mit je 100 Teilen Petroläther verrührt, das Lösungsmittel abdekantiert und das zurückgebliebene, dünnschichtchromatographisch reine Harz wird sorgfältig im Vakuum getrocknet. Das getrocknete, farblosglasige 2-Benzyloxy-1-(4'-chlorphenoxy)-1-[1,2,4-triazolyl-(1)]-3,3-dimethyl-butan (Wirkstoff Nr. 1) zeigt als Isomerengemisch folgende Resonazlinien im $^1H$ NMR-Spektrum (220 MHz, $CDCl_3$): 0.74 u. 0.88 (zwei s, zus. 9H); 3.12, 3.32, 4.08, 4.13, 4.19 u. 4.24 (zus. 3H); 5.56, (s, 1H); 5.93, 5.97, 6.03 und 6.07 (q, 2H); 6.2-6.5 (m, 7H); 6.96 und 7.07 (d, 1H); 7.31 und 7.43 (d, 1H).

Entsprechend können die in der folgenden Tabelle aufgeführten Verbindungen hergestellt werden.

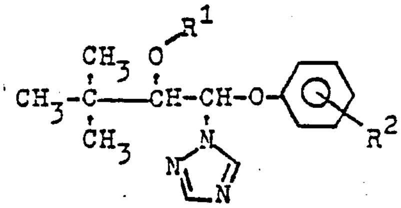

| Wirkstoff Nr. | $R^1$ | $R^2$ | Fp./°C | $^1H$ NMR/δ (ppm) Isomerengemisch |
|---|---|---|---|---|
| 2 | $CH_3$- | 4-Cl | Harz | 270 MHz; $CDCl_3$ 0.87 u. 1.07 (zwei s, zus. 9H); 3.17 u. 3.58 (zwei s, zus. 3H); 3.20 u. 3.53 (zwei s, zus. 1H); 6.3 (d,1H); 6.73 (m,2H); 7.19 (m,2H); 8.0 (d,1H); 8.44 (d,1H). |
| 3 | $C_2H_5$- | 4-Cl | Harz | 220 MHz; $CDCl_3$ 0.9 u. 1.05 (zwei s, zus. 9H); 1.1 (m,3H); 3.4 (m,2H); 6.25 (m,1H); 6.7-7.2 (m,4H); 7.85-7.95 (d,1H); 8.30 u. 8.42 (d,1H). |
| 4 | $CH_2$=CH-$CH_2$- | 4-Cl | Harz | 270 MHz; $CDCl_3$ 0.88 u. 1.07 (zwei s, zus. 9H); 3.44 u. 3.68 (zwei m, zus. 1H); 3.87 u. 4.26 (zwei m, zus. 2H); 5.2 (m,2H); 5.8 (m,1H); 6.31 (s,1H); 6.86 (m,2H); 7.2 (m,2H); 7.98 (d,1H); 8.42 (d,1H). |
| 5 | HC≡C-$CH_2$- | 4-Cl | Harz | 220 MHz, $CDCl_3$ 0.75 u. 0.9 (zwei s, zus 9H); 2.2 (d,1H); 3.2, 3.35, 3.53 u. 3.57 (q,zus.2H); 3.97 (s,1H); 5.58 (d,1H); 6.07 (m,2H); 6.34 (m,2H); 7.05 (d,1H); 7.46 (d,1H). |
| 6 | Cl-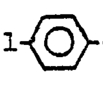-$CH_2$- | 4-Cl | Harz | 270 MHz; $CDCl_3$ 0.91 u. 1.07 (zwei s, zus. 9H); 3.6 (s), 3.84 (d), 4.36 (d) u. 4.73 (q) (zus.3H); 6.34 (s,1H); 6.86 (q,2H); 7.17-7.36 (m,6H); 8.0 (d,1H); 8.36 (d,1H). |

| Wirkstoff Nr. | $R^1$ | $R^2$ | Fp./°C | $^1H$ NMR/δ (ppm) Isomerengemisch |
|---|---|---|---|---|
| 7 | 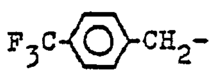 | 4-Cl | Harz | 60 MHz, $CDCl_3$ 1.0 u. 1.2 (zwei s, zus. 9H); 3.62, 3.7, 3.95, 4.12, 4.4, 4.6 u. 4.8 (zus.3H); 6.26 u. 6.33 (d,1H); 6.7, 6.84, 7.1 u. 7.25 (q,4H); 7.35-7.6 (m,4H); 7.88, 8.0, 8.2 u. 8.36 (q,2H). |
| 8 | 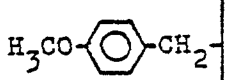 | 4-Cl | Harz | 220 MHz; $CDCl_3$ 0.82 u. 1.02 (zwei s, 9H); 3.68 (s,3H); 6.08 u. 6.15 (d,1H); 6.5-7.1 (m,8H); 7.75 (s,1H); 8.02 (s,1H); |
| 9 | 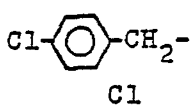 | 4-Cl | Harz | 220 MHz, $CDCl_3$ 0.76 u. 0.91 (zwei s, zus.9H); 3.38, 4.13, 4.19, 4.42 u. 4.48 (zus.3H); 5.58 u. 5.64 (d,1H); 5.97, 6.0, 6.15 u. 6.18 (q,2H); 6.3-6.6 (m,5H); 6.98 u. 7.08 (d,1H); 7.28 u. 7.45 (d,1H). |
| 10 | 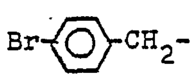 | 4-Cl | Harz | 220 MHz, $CDCl_3$ 0.76 u. 0.9 (zwei s, zus. 9H); 3.13, 3.33, 4.05, 4.09, 4.14 u. 4.19 (zus.3H); 5.57 (s,1H); 5.95, 5.99, 6.03 u. 6.08 (q,2H); 6.2-6.35 (m,4H); 6.5-6.6 (m,2H); 6.97 u. 7.08 (d, 1H); 7.3 u. 7.4 (d,1H). |
| 11 | $CH_3$- (Hydrochlorid) | 2,4-$Cl_2$ | 150-153 | |
| 12 | $CH_2$=CH-$CH_2$- (Hydrochlorid) | 4-Br | 154-157 | |
| 13 | $CH_3$- | 4-Br | | 220 MHz; $CDCl_3$. 0.82 u. 1.02 (zwei s., zus. 9H), 3.13, 3.21, 3.55 u. 3.65 (zus. 4H); 6.26-6.36 (m,1H); 6.72-6.9(m, 2H); 7.28-7.4 (m, 2H); 7.93 u. 8.05 (d, 1H); 8.4 u. 8.5 (d, 1H). |

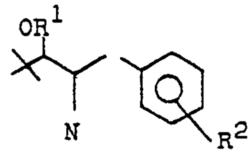

| Wirkstoff Nr. | $R^1$ | $R^2$ | Fp./°C | $^1$H-NMR/δ (ppm) |
|---|---|---|---|---|
| 14 | $CH_3CH_2CH_2$- | 4-Cl | Öl | 100 MHz, $CDCl_3$ (ein Diasteromeres) 0.62 (t, 3H), 1.0 (s, 9H), 1.4 (m, 2H), 2.8 und 3.2 (m, 2H), 3,3 (d, 1H) 6.2 (d, 1H), 6.75 (d, 2H) 7.15 (d, 2H), 7.93 (s, 1H) und 8.26 (s, 1H). |
| 15 | $CH_3CH_2CH_2CH_2$- | 4-Cl | 62-63 | |
| 16 | $CH_3(CH_2)_4$- | 4-Cl | Öl | |
| 17 | $CH_3$-CH=CH-$CH_2$- | 4-Cl | Öl | 270 MHz, $CDCl_3$ x) 0.88 u. 1.08 (zwei s, 9H), 1.7 (zwei d, 3 H), 6.3 (zwei d, 1H), 6.88 (m, 2H) 7.15 (m, 2H), 7.94 und 8.2 (d, 1H), 8.38 und 8.52 (d, 1H) |
| 18 | $H_2C=C(CH_3)-CH_2$- | 4-Cl | Öl | 220 MHz, $CDCl_3$ x) 0.85 und 1.02 (zwei s, 9H), 1.69 und 1.78 (zwei s, 3H), 8.03 und 8.5 (zwei d, je 1H) |
| 19 | $H_3C-C≡C-CH_2$- | 4-Cl | Öl | 270 MHz, $CDCl_3$ (ein Diastereomeres) x) 0.86 (s, 9H), 1.88 (s, 3H) 7.95 und 8.56 zwei s, je 1H) |
| 20 | 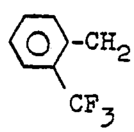 | 4-Cl | Öl | 100 MHz, $CDCl_3$ x) 0.88 und 1.02 (zwei s, 9H), 7.86 und 8.24 (zwei d, je 1H) |
| 21 | 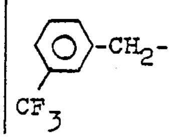 | 4-Cl | Öl | 100 MHz, $CDCl_3$ x) 0.92 und 1.06 (zwei s, 9H), 7.95 und 8.32 (zwei d, je 1H) |

x) nur die charakteristischen Signale sind angegeben

| Wirkstoff Nr. | $R^1$ | $R^2$ | Fp./°C | $^1$H-NMR/δ (ppm) |
|---|---|---|---|---|
| 22 | 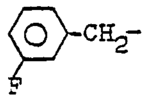 | 4-Cl | Öl | 220 MHz, $CDCl_3$ ⁎) 0.86 und 1.02 (zwei s, 9H), 8.0 und 8.38 (zwei d, je 1H) |
| 23 | 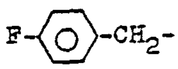 | 4-Cl | Öl | 220 MHz, $CDCl_3$ ⁎) 0.86 und 1.02 (zwei s, 9H), 8.0 und 8.38 (zwei d, je 1H) |
| 24 | 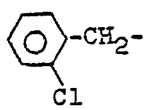 | 4-Cl | Öl | 220 MHz, $CDCl_3$ ⁎) 0.86 und 1.02 (zwei s, 9H), 8.02 und 8.4 (zwei d, je 1H) |
| 25 | 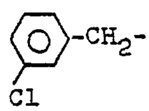 | 4-Cl | Öl | 220 MHz, $CDCl_3$ ⁎) 0.88 und 1.03 (zwei s, 9H), und 8.0 und 8.38 (zwei d, je 1H) |
| 26 | 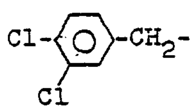 | 4-Cl | Öl | |
| 27 | 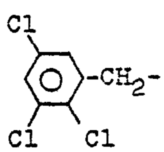 | 4-Cl | Öl | 270 MHz, $CDCl_3$ ⁎) 0.84 und 0.98 (zwei s, 9H), 7.94 und 8.4 (zwei d, je 1H) |
| 28 | 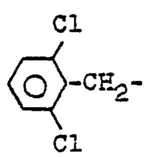 | 4-Cl | Öl | |

⁎) nur die charakteristischen Signale sind angegeben.

Die erfindungsgemäßen Verbindungen und ihre Salze und Metallkomplexverbindungen zeichnen sich durch eine hervorragende Wirksamkeit gegen ein breites Spektrum von Pilzen, speziell pflanzenpathogenen Pilzen, insbesondere aus der Klasse der Ascomyceten und Basidiomyceten, aus. Sie sind zum Teil systemisch und können als Blatt- und Bodenfungizide, besonders aber auch als Beizmittel, eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen verstehen wir in diesem Zusammenhang insbesondere Weizen, Roggen, Gerste, Hafer, Reis, Mais, Baumwolle, Soja, Kaffee, Zuckerrohr, Obst und Zierpflanzen im Gartenbau, sowie Gemüse wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Hemileia vastatrix an Kaffee,
Uromyces phaseoli an Bohnen,
Ustilage nuda an Gerste,
Ustilago tritici und Tilletia tritici an Weizen.

Die neuen Wirkstoffe eignen sich ferner zur Anwendung im Materialschutz, insbesondere zum Schutz von Holz und Holzwerkstoffen, gegen die Zerstörung durch Pilze aus der Klasse der Basidiomyceten. Im einzelnen sind folgende Pilze zu nennen: Coniophora puteana, Merulius lacrymans, Poria monticola, Lenzites trabea, Lenzites abietina, Trametes versicolor, Armillaria mellea, Stereum purpureum, Fomes annosus.

Die erfindungsgemäßen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken, sie sollen in jedem Fall eine feine und gleichmäßig Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürliche Gesteinsmehlte (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyäthylen - Fettalkohol - Äther, Alkylsulfonate und Arylsulfonate) und Dispergiermittel, wie Lignin-, Sulfitablaugen und Methylcellulose.

Die fungiziden Mittel enthalten im allgemeinen zwischen 0,1 und 95 Gewichtsprozent Wirkstoff, vorzugsweise zwischen 0,5 und 90.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die Aufwandmengen liegen nach Art des gewünschten Effektes zwischen 0,01 und 3 oder mehr, vorzugsweise jedoch zwischen 0,01 und 1 kg Wirkstoff pro Hektar.

Die Wirkstoffe können auch zusammen mit anderen Wirkstoffen, z.B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die deer addierten Wirksamkeiten der Einzelkomponenten.

Die folgende Liste von Fungiziden, mit denen die Wirkstoffe kombiniert werden können, soll die Kombinationsmöglichkeiten erläutern, nicht aber einschränken.

Fungizide, die mit den Wirkstoffen kombiniert werden können, sind beispielsweise:

Dithiocarbamate und deren Derivate, wie
Ferridimethyldithiocarbamat,
Zinkdimethyldithiocarbamat,
Manganäthylenbisdithiocarbamat,
Mangan-Zink-äthylendiamin-bis-dithiocarbamat,

Zinkäthylenbisdithiocarbamat,
Tetramethylthiuramdisulfide,
Ammoniak-Komplex von Zink-(N,N-äthylen-bis-dithiocarbamat) und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diäthyl-phthalimidophosphonothioat,
5-Amino-1-(bis-(dimethylamino)-phosphinyl)-3-phenyl-1,2,4-triazol,
5-Äthoxy-3-trichloromethyl-1,2,4-thiadiazol,
2,3-Dicyano-1,4-dithiaanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid
2,3-Dihydro-5-carboxanilido-6-methyl-1,4-oxathiin,

2-(Furyl-(2))-benzimidazol,
Piperazin-1,4-diyl-bis-(1-(2,2,2-trichlor-äthyl)-formamid,
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-äthoxycarbonyl-2-thioureido)-benzol,
1,2-Bis-(3-methoxycarbonyl-2-thioureido)-benzol
und verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäure-diamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze,
N-[3-(p-tert.Butyl-phenyl)-2-methyl-propyl]-cis-2,6-dimethyl-morpholin,
N-[3-(p-tert.Butyl-phenyl)-2-methyl-propyl]-3,5-dimethyl-piperidin,
N-[3-(p-tert.Butyl-phenyl)-2-methyl-propyl]-piperidin,
Methyl-N-(2,6-dimethyl-phenyl)-N-(2-furoyl)-alaninat,
Methyl-N-(2,6-dimethyl-phenyl)-N-(2-methoxyacetyl)-alaninat,
N-(2,6-Dimethyl-phenyl)-N-[2-oxo-tetrahydrofuranyl-(3)]-chloracetamid,
2-Cyano-N-ethylaminocarbonyl-2-methoximino-acetamid,
4-N-Butyl-1,2,4-triazol,
5-Nitro-isophthalsäure-diisopropylester,
3-(3,5-Dichlorphenyl)-5-methyl-5-ethenyl-2,4-oxazolidin-dion,

3-(3,5-Dichlorphenyl)-2,4-dioxo-1-(N-isopropyl)-imidazolidincarboxamid,
1,2-Dimethyl-cyclopropandicarbonsäure-N-(3',5'-dichlorphenyl)-imid;
Aluminiumsalz a des N'-Hydroxy-N-cyclohexyl-diazeniumoxids,
N-Methoxy-2,5-dimethyl-furan-3-carbonsäurecyclohexylamid.

Die folgenden Beispiele erläutern die fungizide Wirkung der neuen Wirkstoffe.

Als Vergleichssubstanz wurde die in der DE-OS 20 63 857 beschriebene Verbindung der Formel

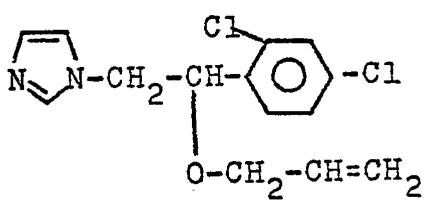 A,

die im folgenden der Einfachheit halber mit A bezeichnet wird, verwendet.

Beispiel 2

Blätter von in Töpfen gewachsenen Weizenkeimlingen der Sorte "Jubilar" werden mit wäßrigen Emulsionen aus 80 % (Gewichtsprozent) Wirkstoff und 20 % Emulgiermittel besprüht und nach dem Antrockmen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff Nr. | Befall der Blätter nach Spritzungen mit x %iger Wirkstoffbrühe x = 0,006 | 0,012 | 0,025 |
|---|---|---|---|
| 2 | 0 | 0 | 0 |
| 4 | 0 | 0 | 0 |
| 5 | 0 | 0 | 0 |
| 6 | 1 | 0 | 0 |
| A | 4 | 3 | 3 |
| Kontrolle (unbehandelt) | 5 | | |

0 kein Befall, abgestuft bis 5 Totalbefall

Beispiel 3

In einem weiten Versuch werden, wie im vorhergehenden Beispiel beschrieben, Blätter von in Töpfen gewachsenen Gerstenkeimlingen der Sorte "Firlbecks Union" behandelt und mit Sporen (Konidien) des Gerstenmehltaus (Erysiphe graminis var. hordei) bestäubt.

| Wirkstoff Nr. | Befall der Blätter nach Spritzungen mit x %iger Wirkstoffbrühe x = 0,006 | 0,012 | 0,025 |
|---|---|---|---|
| 2 | 0 | 0 | 0 |
| 4 | 0 | 0 | 0 |
| 5 | 0 | 0 | 0 |
| 6 | 0 | 0 | 0 |
| A | 2 | 1 | 0 |
| Kontrolle (unbehandelt) | 5 | | |

Beispiel 4

Blätter von in Töpfen gewachsenen Weizensämlingen der Sorte "Caribo" werden mit Sporen des Braunrostes (Puccinia recondita)

bestäubt. Danach werden die Töpfe für 24 Stunden bei 20 bis 22°C in eine Kammer mit hoher Luftfeuchtigkeit (90-95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Ligninsulfonat in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blättern ermittelt.

| Wirkstoff Nr. | Befall der Blätter nach Spritzungen mit x %iger Wirkstoffbrühe x = 0,006 | 0,012 | 0,025 |
|---|---|---|---|
| 2 | 0 | 0 | 0 |
| 4 | 0 | 0 | 0 |
| 6 | 1 | 1 | 0 |
| A | 5 | 5 | 4 |
| Kontrolle (unbehandelt) | 5 | | |

Beispiel 5

100 g-Proben Gerstensaatgut der Sorte "Asse" werden in Glasflaschen etwa 5 Minuten lang mit jeweils 300 mg (= 0,3 Gew.%) der in der Tabelle angeführten Beizmittel sorgfältig gebeizt. Danach werden jeweils 8 Körper in Töpfe eingelegt und mit Erde bedeckt. Zehn Tage nach dem Auflauf des Getreides werden die Blätter mit Oidium (Konidien) des Gerstenmehltaus (Erysiphe graminis var. hordei) bestäubt. Die Versuchspflanzen werden anschließend im Gewächshaus bei Temperaturen zwischen 20 und 22°C und 65-70 % relativer Luftfeuchte aufgestellt. Nach weiteren 10 Tagen wird das Ausmaß der Mehltaupilzentwicklung auf den Blättern ermittelt.

| Wirkstoff Nr. | ... % Wirkstoff im Beizmittel | Ausmaß des Mehltaubefalls auf den Blättern 10 Tage nach künstl. Infektion |
|---|---|---|
| 2 | 10 | 0 |
| 5 | 10 | 0 |
| 6 | 10 | 0 |
| Kontrolle (unbehandelt) | - | 5 |

Beispiel 6

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 7

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteile des Anlagerungsproduktes von 40 Mol Äthylenoxid an 1 Mol Rizinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

Beispiel 8

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsprodukte von 40 Mol Äthy-

lenoxid an 1 Mol Rizinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 9

20 Gewichtsteile der Verbindung 1 werden in einer Mischung gelöst, die aus 25 Gewichtsteilen Cyclohexanol, 65 Gewicht teilen einer Mineralölfraktion vom Siedepunkt 210 bis 280°C und 10 Gewichtsteilen des Anlagerungsproduktes von 40 Mol Äthylenoxid an 1 Mol Rizinusöl besteht. Durch Eingießen und feines Verteilen des Lösung in 100 000 Gewichts teilen Wasser erhält man eine wäßrige Dispersion, die 0,02 Gewichtsprozent des Wirkstoffs enthält.

Beispiel 10

20 Gewichtsteile des Wirkstoffs 2 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 11

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 12

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

Beispiel 13

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 14

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Beispiel 15

Zur Herstellung eines öligen Holzschutzmittel mit 0,5 % (Gew.%) 2-Methoxy-1-(4'-chlorphenoxy)-1-[1,2,4-triazolyl-(1)]-3,3-dimethyl-butan (Wirkstoff 2) werden 0,5 Teile des Wirkstoffs mit 15 Teilen eines Alkydharzes mit mittlerem Gehalt an Ölen (20 % Festharz) gemischt. Anschlie-

ßend fügt man 45 Teile einer aromatenhaltigen Benzinfraktion hinzu, filtriert gegebenenfalls, um Verunreinigungen zu beseitigen, und füllt mit einer aliphatenhaltigen Benzinfraktion auf 100 Teile auf.

In analoger Weise werden 1 und 2 %ige ölige Holzschutzmittel mit dem Wirkstoff 2 hergestellt.

Beispiel 16

Zur Ermittlung der fungiziden Wirksamkeit gegenüber den Pilzen Coniophora puteana und Trametes versicolor wurden Kiefernsplintholzklötzchen mit den Abmessungen 50 x 25 x 15 mm mit öligen Holzschutzmittelzubereitungen, die 0,5, 1 und 2 % Wirkstoff enthielten in Mengen von jeweils 200 $g/m^2$ Holzoberfläche bestrichen. Nach vierwöchiger Lagerung wurden die behandelten Klötzchen zusammen mit den unbehandelten in Glasschalen gelegt, die als Prüfpilz Coniophora puteana bzw. Trametes versicolor auf einem Nähragar enthielten. Die Schalen wurden anschließend in einem Klimaraum bei einer Temperatur von 22°C und einer relativen Luftfeuchte von 70 % bebrütet. Nach dreimonatiger Versuchsdauer wurden die Klötzchen von anhaftendem Pilzmycel befreit und getrocknet. Anschließend wurde das Ausmaß der Holzzerstörung festgestellt.

| Wirkstoff | ... % Wirkstoff in der Zubereitung | Ausmaß des Pilzangriffs nach dreimonatiger Versuchsdauer Coniophora puteana | Trametes versicolor |
|---|---|---|---|
| 2 | 0,5 | 1 | 1 |
| | 1 | 1 | 1 |
| | 2 | 1 | 1 |
| 4 | 0,5 | 1 | 1 |
| | 1 | 1 | 1 |
| | 2 | 1 | 1 |
| Pentachlorphenol (bekannt) | 1 | 3a | 3a |
| | 2 | 2b/3a | 2b |
| Kontrolle (nur Alkydharz-Lösungsmittel, ohne Wirkstoff) | - | 3a/4b | 3 b |

Bewertungsschema:
1 unversehrt
2a stellenweise wenig angegriffen
2b im ganzen wenig angegriffen
3a stellenweise stark angegriffen
3b im ganzen stark angegriffen
4a stellenweise völlig zerstört
4b im ganzen völlig zerstört

Patentansprüche

1. Triazolyl-glykoläther der Formel

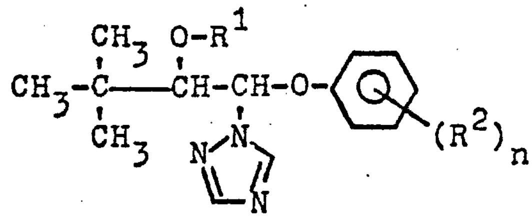

in der

$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluormethyl substituiert ist,

$R^2$ ein Halogenatom bedeutet und

n 1 oder 2 bedeutet

und deren Salze sowie Metallkomplexverbindungen.

2. Fungizid, enthaltend einen Triazolyl-glykoläther der Formel

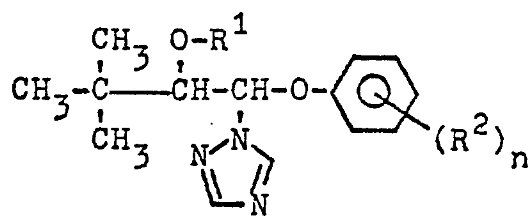

in der

$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluor-methyl substituiert ist,

$R^2$ ein Halogenatom bedeutet und
n 1 oder 2 bedeutet
und deren Salze sowie Metallkomplexverbindungen.

3. Verfahren zur Bekämpfung von Pilzen, dadurch gekennzeichnet, daß man die vor Pilzbefall zu schützenden Pflanzen oder die Pilze behandelt mit einem Triazol-glykoläther der Formel

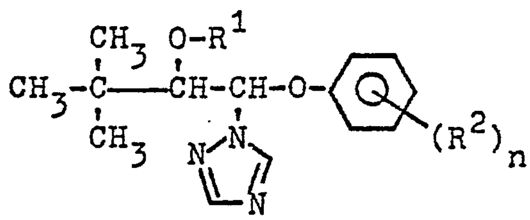

in der
$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluormethyl substituiert ist,
$R^2$ ein Halogenatom bedeutet und
n 1 oder 2 bedeutet
und deren Salze sowie Metallkomplexverbindungen.

4. Verfahren zur Herstellung von Triazolyl-glykoläthern der Formel

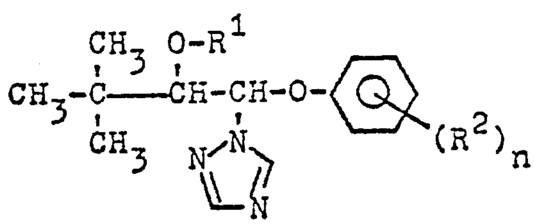

in der

$R^1$ einen Alkylrest mit bis zu 5 C-Atomen, einen Alkenylrest mit 3 bis 5 C-Atomen, einen Alkinylrest mit 3 bis 5 C-Atomen oder einen Benzylrest bedeutet, der gegebenenfalls durch Halogen, Alkoxy oder Trifluormethyl substituiert ist,

$R^2$ ein Halogenatom bedeutet und

n 1 oder 2 bedeutet

und deren Salze sowie Metallkomplexverbindungen, dadurch gekennzeichnet, daß man ein Triazolylderivat der Formel

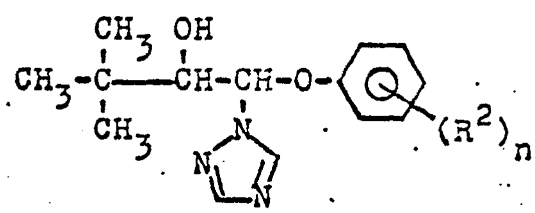

in der $R^2$ und n die obengenannte Bedeutung haben, mit einem Halogenderivat der Formel

$$Hal\text{-}R^1$$

in der $R^1$ die im Anspruch 1 genannte Bedeutung hat und Hal Halogen, insbesondere Chlor, Brom oder Jod bedeutet, in Gegenwart eines Lösungsmittels bei einer Temperatur im Bereich von -80 bis +80°C umsetzt und diese Verbindung gegebenenfalls mit einer Säure in ihr Salz oder mit einem Metallsalz in ihre Metallkomplexverbindung überführt.

5. Triazol-glykoläther, ausgewählt aus der Gruppe bestehend aus

2-Methoxy-1-(4'-chlorphenoxy)-1-[1,2,4-triazolyl-(1)]-3,3-dimethyl-butan

2-Äthoxy-1-(4'-chlorphenoxy)-1-[1,2,4-triazolyl-(1)]-3,3-dimethyl-butan

2-Allyloxy-1-(4'-chlorphenoxy)-1-[1,2,4-triazolyl-(1)]-3,3-dimethyl-butan

2-Propargyloxy-1-(4'-chlorphenoxy)-1-[1,2,4-triazolyl-(1)] - 3,3,dimethyl-butan

2-(4'-Chlorbenzyloxy)-1-(4"-chlorphenoxy)-1-[1,2,4-triazolyl-(1)]-3,3-dimethyl-butan

2-(4'-Trifluormethyl-benzyloxy)-1-(4"-chlorphenoxy)-1-[1,2,4-triazolyl-(1)]-3,3-dimethyl-butan

2-(4'-Methoxy-benzyloxy)-1-(4"-chlorphenoxy)-1-[1,2,4-triazolyl-(1)]-3,3-dimethyl-butan

2-(2',4'-Dichlor-benzyloxy)-1-(4"-chlorphenoxy)-1-[1,2,4-triazolyl-(1)]-3,3-dimethyl-butan

2-(4'-Brombenzyloxy)-1-(4"-chlorphenoxy)-1-[1,2,4-triazolyl-(1)]-3,3-dimethyl-butan

2-Benzyloxy-1-(4'-chlorphenoxy)-1-[1,2,4-triazolyl-(1)]-3,3-dimethyl-butan

2-Methoxy-1-(4'-bromophenoxy)-1-[1,2,4-triazolyl-(1)]-3,3-dimethyl-butan

2-Allyloxy-1-(4'-bromphenoxy)-1-[1,2,4-triazolyl-(1)]-3,3-dimethyl-butan

2-Methoxy-1-(2',4'-dichlorphenoxy)-1-[1,2,4-triazolyl-(1)]-3,3-dimethyl-butan.

6. Fungizid, enthaltend einen Triazolyl-glykoläther, ausgewählt aus der Gruppe bestehend aus

2-Methoxy-1-(4'-chlorphenoxy)-1-[1,2,4-triazolyl-(1)]-3,3-dimethyl-butan

2-Äthoxy-1-(4'-chlorphenoxy)-1-[1,2,4-triazolyl-(1)]-3,3-dimethyl-butan

2-Allyloxy-1-(4'-chlorphenoxy)-1-[1,2,4-triazolyl-(1)]-3,3-dimethyl-butan

2-Propargyloxy-1-(4'-chlorphenoxy)-1-[1,2,4-triazolyl-(1)]-3,3-dimethyl-butan

2-(4'-Chlorbenzyloxy)-1-(4"-chlorphenoxy)-1-[1,2,4-triazolyl-(1)]-3,3-dimethyl-butan

2-(4'-Trifluormethyl-benzyloxy)-1-(4"-chlorphenoxy)-1-[1,2,4-triazolyl-(1)]-3,3-dimethyl-butan

2-(4'-Methoxy-benzyloxy)-1-(4"-chlorphenoxy)-1-[1,2,4-triazolyl-(1)]-3,3-dimethyl-butan

2-(2',4'-Dichlor-benzyloxy)-1-(4"-chlorphenoxy)-1-[1,2,4-triazolyl-(1)]-3,3-dimethyl-butan

2-(4'-Brombenzyloxy)-1-(4"-chlorphenoxy)-1-[1,2,4-triazolyl-(1)]-3,3-dimethyl-butan

2-Benzyloxy-1-(4'-chlorphenoxy)-1-[1,2,4-triazolyl-(1)]-3,3-dimethyl-butan

2-Methoxy-1-(4'-bromophenoxy)-1-[1,2,4-triazolyl-(1)]-3,3-dimethyl-butan

2-Allyloxy-1-(4'-bromphenoxy)-1-[1,2,4-triazolyl-(1)]-3,3-dimethyl-butan

2-Methoxy-1-(2',4'-dichlorphenoxy)-1-[1,2,4-triazolyl-(1)]-3,3-dimethyl-butan.

Europäisches Patentamt

# EUROPÄISCHER RECHERCHENBERICHT

0002671

Nummer der Anmeldung

EP 78 10 1486

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 600 799 (BAYER)<br>* Patentansprüche *<br>-- | 1,2,3,4 |
| XP | DE - A - 2 720 949 (BAYER)<br>* Patentansprüche, Seiten 1,2,3; Beispiele 1,2,5,20,9,13,29, Seiten 49-57 *<br>---- | 1,2,3,4,5,6 |

KLASSIFIKATION DER ANMELDUNG (Int.Cl.²)

C 07 D 249/08
A 01 N 9/22

RECHERCHIERTE SACHGEBIETE (Int. Cl.²)

C 07 D 249/08

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 29-03-1979 | CREMERS |